Europäisches Patentamt
European Patent Office
Office européen des brevets

(19) (11) Publication number: **0 013 620 A1**

(12) **EUROPEAN PATENT APPLICATION**

(21) Application number: 80300080.1

(22) Date of filing: 08.01.80

(51) Int. Cl.[3]: C 07 C 45/65
C 07 C 49/707, C 07 C 45/67
C 07 F 7/18
//C07C69/145, C07C69/78

(30) Priority: 11.01.79 AU 7328/79

(43) Date of publication of application:
23.07.80 Bulletin 80/15

(84) Designated Contracting States:
AT BE CH DE FR GB IT LU NL SE

(71) Applicant: THE AUSTRALIAN NATIONAL UNIVERSITY
Acton Australian Capital Territory(AU)

(72) Inventor: Rickards, Rodney Warren
3 Davis Street
Weetangera Australian Capital Territory(AU)

(72) Inventor: Gill, Melvyn
14 Carroll Street
Hughes Australian Capital Territory(AU)

(74) Representative: Day, Jeremy John et al,
REDDIE & GROSE 16 Theobalds Road
London, WC1X 8PL(GB)

(54) **Process for the peparation of 4-hydroxycyclopent-2-enone and derivatives.**

(57) A process for the preparation of compounds of the general formula I:

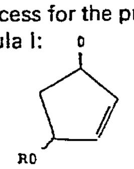

I

wherein R represents hydrogen or a protecting group, which comprises selective reduction of a compound of the general formula II:

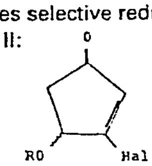

II

wherein R is as defined above, and Hal represents halogen; with optional total or partial inversion of the hydroxyl or protected hydroxyl substituent; and, when R is a protecting group, optional removal of the protecting group from the compound of general formula I.

EP 0 013 620 A1

"PROCESS FOR THE PREPARATION OF 4-HYDROXYCYCLOPENT-2-ENONE AND DERIVATIVES"

This invention relates to a process for the synthetic preparation of 4-hydroxycyclopent-2-enone and derivatives of this compound in which the hydroxyl function is protected, particularly in the chiral (4 R)- and (4 S)- enantiomeric forms. These compounds are of importance, for example as intermediates in the synthesis of compounds of the prostaglandin group, including both naturally-occurring members and various modified analogues, herein collectively termed prostanoids.

The natural prostaglandins are structurally-related lipids which are normally present at very low concentrations in human and animal tissues, or are elaborated by these tissues in response to certain stimuli. They are believed to be involved in the control of a wide variety of biological processes, including reproduction, muscle expansion and contraction, respiration, lipid metabolism, kidney function, central nervous system activity, gastric secretion, cardiovascular activity, immune response, and temperature control. Their chemistry and biology have been the subjects of extensive research and review. (See for example, P. Crabbé (ed.), "Prostaglandin Research", Academic Press, New York, 1977; K.H. Gibson, Chem. Soc. Rev., 1977, 6, 489; N. Kharasch and J. Fried (eds.), "Biochemical Aspects of Prostaglandins and Thromboxanes", Academic Press, New York, 1977).

A number of routes are available for the laboratory synthesis of both the natural prostaglandins themselves and also modified analogues of the natural prostaglandins. (See for example, A. Mitra, "The Synthesis of Prostaglandins", Wiley and Sons, New York, 1977; J.S. Bindra and R. Bindra, "Prostaglandin Synthesis", Academic Press, New York, 1977; P. Crabbé (ed.), "Prostaglandin Research", Academic Press, New York, 1977; D. Orth and H.-E. Radunz, Top. Curr. Chem., 1977, 72, 51). Modified analogues in some cases offer improved stability towards metabolic inactivation in the

body, and/or improved selectivity of biological action, relative to the natural prostaglandins.

One important synthetic route to prostanoids involves the conjugate addition of organometallic reagents to derivatives of 4-hydroxycyclopent-2-enone in which the hydroxyl function is protected with a suitable protecting group (see above references). This leads to β-alkylated enolates, in which the alkyl substituent will ultimately provide the β-chain of the prostanoid. Reaction of these enolates with electrophiles permits the introduction of the eventual α-chain of the final prostanoids. Thus, for example, the cumyl ether (G. Stork and M. Isobe, J. Am. Chem. Soc., 1975, 97, 6260; M.J. Loots and J. Schwartz, Tetrahedron Lett., 1978, 4381), the t-butyldimethylsilyl ether (T. Tanaka, S. Kurozumi, T. Toru, M. Kobayashi, S. Miura, and S. Ishimoto, Tetrahedron, 1977, 33, 1105; R. Davis and K.G. Untch, J. Org. Chem., 1979, 44, 3755), the 2-chloroethyl ether (J.W. Patterson and J.H. Fried, U.S. Pat., 1974, 3,847,962, cf. Chem. Abstr., 1975, 82, 86120s; U.S. Pat., 1975, 3,872,139, cf. Chem. Abstr., 1975, 83, 131204f), and alkoxy-alkyl ethers (J.W. Patterson and J.H. Fried, U.S. Pat., 1975, 3,872,139, cf. Chem. Abstr., 1975, 83, 131204f) of racemic 4-hydroxycyclopent-2-enone, and the t-butyldimethylsilyl ether of the (4R)-enantiomer of 4-hydroxycyclopent-2-enone (T. Tanaka, S. Kurozumi, T. Toru, M. Kobayashi, S. Miura, and S. Ishimoto, Tetrahedron, 1977, 33, 1105) have been used in this way for the synthesis of various prostanoids,

Significantly, the prostanoids resulting from this route possess the required trans, trans relative configuration of the three substituents on the cyclopentanone ring. Furthermore, use of the chiral (4R)-enantiomers of the 4-hydroxycyclopent-2-enone derivatives leads to prostanoids in which these three ring substituents

have the same absolute configuration as in natural prostaglandins.

Chiral 4-hydroxycyclopent-2-enones have previously been prepared by a combination of chemical and microbiological processes, but the desired (4R)- enantiomers which result only in low overall yield are not fully optically pure, being present in up to 90% enantiomeric excess (T. Tanaka, S. Kurozumi, T. Toru, S. Miura, M. Kobayashi, and S. Ishimoto, Tetrahedron, 1976, 32, 1713, see also S. Kurozumi et. al. U.S. Patent No. 4132726). Alternatively, chiral 4-hydroxycyclopent-2-enones have been prepared by chemical modification of chiral substrates such as (2R, 3R)- and (2S, 3S)-tartaric acid (K. Ogura, M. Yamashita, and G. Tsuchihashi, Tetrahedron Lett., 1976, 759; see also G. Tsuchihashi, et. al. Japan Kokai 76-95042) and terrein (L.A. Mitscher, G.W. Clark, and P.B. Hudson, Tetrahedron Lett., 1978, 2553), but these routes also have disadvantages. In the case of tartaric acid, the resulting chiral 4-hydroxycyclopent-2-enones are again only 85-86% optically pure, and the desired (4R)-enantiomers are derived from the less common (2S, 3S)- or D-(-)-tartaric acid. Terrein yields the acetate ester of the desired (4R)-4-hydroxycyclopent-2-enone optically pure, but is itself a complex metabolite available only from fermentation of certain fungi.

It is an object of the present invention to provide an improved process for the preparation of 4-hydroxycyclopent-2-enone and derivatives thereof in which the hydroxyl function is protected, particularly in the chiral (4R)- and (4S)-enantiomeric forms.

According to the present invention, there is provided a process for the preparation of compounds of the general formula I :

 

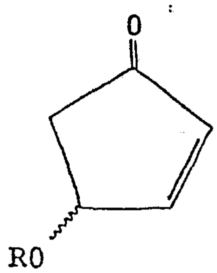

I

[of which formulae Ia and Ib represent the enantiomeric forms thereof :

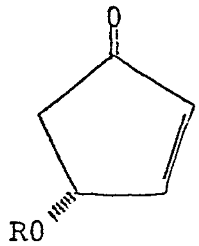 Ia 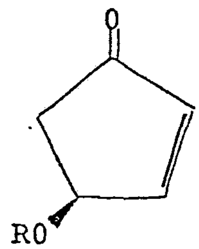 Ib ]

wherein R represents hydrogen or a protecting group, which comprises the selective reduction of compounds of the general formula II :

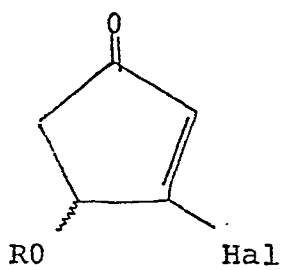

II

[of which formulae IIa and IIb represent the enantiomeric forms thereof :

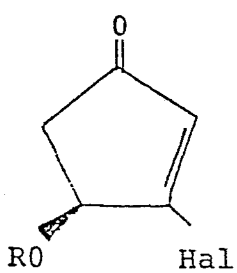 IIa 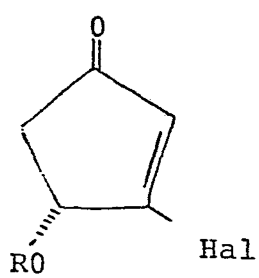 IIb ]

wherein R is as defined above, and Hal represents

halogen, particularly chlorine, bromine or iodine, and, if desired, total or partial inversion of the hydroxyl or protected hydroxyl substituent.

As used throughout this specification, the term "protecting group" is used to denote a removable protective group for an alcoholic hydroxyl group. Such groups are well known. Examples of groups which protect as ethers include, for example, substituted alkyl groups such as alkoxyalkyl groups, tetrahydrofuran-2-yl and tetrahydropyran-2-yl groups, and substituted silyl groups such as silyl groups trisubstituted with alkyl and/or aryl residues, for example, the t-butyldimethylsilyl group. Examples of groups which protect as esters include derivatives of alkyl or aryl carboxylic acids, for example, acetyl and benzyl groups.

It will be appreciated that the compounds of the general formulae I and II referred to throughout this specification are chiral and the present invention relates both to the individual enantiomers and to any mixtures thereof, whether these mixtures be racemic or partially racemic. In accordance with accepted nomenclature, the dotted lines used in the formulae throughout this specification indicate that the attached group lies behind the general plane of the ring system, i.e., that the group is in an α-configuration; whilst the thickened lines indicate that the attached group lies in front of the general plane of the ring system, i.e., that the group is in a β-configuration. The wavy lines used in the formulae throughout this specification indicate that the attached group is present in an α- or β-configuration or is present in both α- and β-configurations, thus including not only both individual enantiomers thereof but also all mixtures of such enantiomers.

As noted above, various prostanoids have been prepared from compounds of the general formula I. If desired, the

protecting group in these compounds may be removed or replaced by an alternative protecting group prior to conversion of the compound of general formula I into such prostanoids. In addition, a racemic mixture of a compound of the general formula I may, if desired, be totally or partially resolved. Such resolution may be effected by known methods.

Formulae (1) to (8) referred to in the following detailed description of an example of the process of the present invention are set out on the following page.

It will be appreciated from the above general description and from the further description which follows that the present invention offers advantages in the preparation of 4-hydroxycyclopent-2-enone (1; R=H) and its derivatives (1; R=protecting group), particularly in either enantiomeric form (2; R=H or protecting group) or (3; R=H or protecting group). These advantages include the use of a cheap, readily available starting material, and its conversion by efficient, convenient, non-microbiological processes into the required optically pure enantiomers.

The preparation of (4S)-3-chloro-4-hydroxycyclopent-2-enone (5; R=H) and its ethers (5; R=protecting group) exemplified by its t-butyldimethylsilyl ether (5; $R=SiBu^tMe_2$) from phenol or 2,4,6-trichlorophenol has been described in copending European Patent Application No. 79301822.7 (see also M. Gill and R.W. Rickards, J. Chem. Soc. Chem. Comm., 1979, 121).

In this preparation, the benzenoid ring undergoes contraction to (1R*, 4R*)-3,5,5,-trichloro-1,4-dihydroxycyclopent-2-ene-1-carboxylic acid, and chirality is introduced by resolution of this racemic acid into its (1R, 4R)- and (1S, 4S)-enantiomers (7) and (8) respectively. The (1R, 4R)-enantiomer (7) is then oxidatively decarboxylated and partially dechlorinated to yield (4S)-3-chloro-

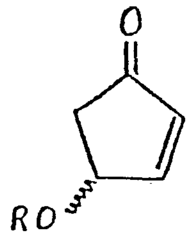

(1)

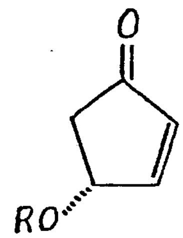

(2)

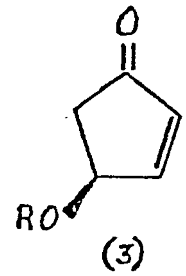

(3)

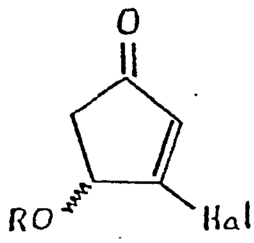

(4)

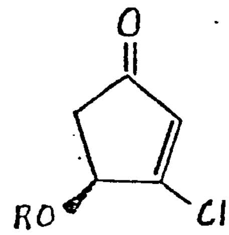

(5)

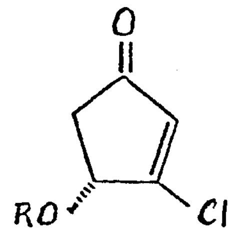

(6)

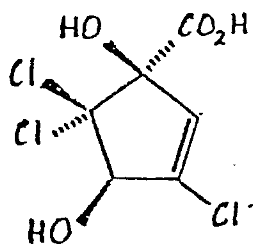

(7)

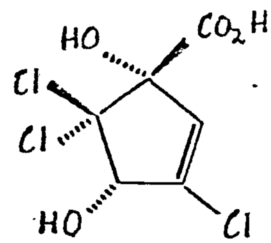

(8)

4-hydroxycyclopent-2-enone (5; R=H), derivatisation then affording the (4S)-ethers (5;R=protecting group).

The preparation of the enantiomeric (4R)-3-chloro-4-hydroxycyclopent-2-enone (6; R=H) and its ethers (6; R=protecting group),exemplified by its t-butyldimethylsilyl ether (6; R=$SiBu^tMe_2$)by a similar process from the (1S, 4S)-enantiomer (8) of the 3,5,5-trichloro-1,4-dihydroxycyclopent-2-ene-1-carboxylic acid has also been described in copending European Application No. 79301822.7.

These (4S)- and (4R)-enantiomers of 3-chloro-4-hydroxycyclopent-2-enone (5 and 6; R=H) can be selectively reduced by suitable reagents, for example bimetallic couples, to the corresponding (4S)- and (4R)-enantiomers of 4-hydroxycyclopent-2-enone (3 and 2; R=H). Alternatively, the hydroxyl functions in the (4S)- and (4R)-enantiomers (5 and 6; R=H) can be protected as suitable derivatives (5 and 6; R=protecting group), for example as the substituted alkyl or trisubstituted silyl ethers, before reduction to the corresponding derivatives (3 and 2; R=protecting group) of the (4S)- and (4R)-enantiomers of 4-hydroxycyclopent-2-enone. The protecting groups in the reduction products ( 3 and 2; R=protecting group) can then be removed if desired to yield the corresponding (4S)- and (4R)-enantiomers of 4-hydroxycyclopent-2-enone (3 and 2; R=H).

By these processes described, phenol can be converted via the (1R, 4R)-3,5,5-trichloro-1,4-dihydroxycyclopent-2-ene-1-carboxylic acid (7) into (4S)-4-hydroxycyclopent-2-enone (3; R=H) and its derivatives (3; R=protecting group), or via the enantiomeric (1S, 4S)-acid (8) into (4R)-4-hydroxycyclopent-2-enone (2; R=H) and its derivatives (2; R=protecting group). However, (4S)-4-hydroxycyclopent-2-enone (3; R=H) and its derivatives (3; R=protecting group) can also be prepared from the (1S, 4S)-acid (8) by carrying out an inversion of configuration of

the secondary hydroxyl or protected secondary hydroxyl centre. Similarly, (4R)-4-hydroxycyclopent-2-enone (2; R=H) and its derivatives (2; R=protecting group) can also be prepared from the (1R, 4R)-acid (7). It will be appreciated that such inversion may be effected at any appropriate stage of the synthetic sequence, either before or after the selective reduction of the compounds of the general formula II to compounds of the general formula I. Bimolecular nucleophilic substitution reactions are suitable for this inversion.

Specific details of the reactions involved in the processes of this invention are illustrated by the following detailed examples. In these examples, all temperatures are in degrees Centigrade, and technical terms (e.g. chromatography, etc.,) have the usual meaning in the art. Crude reaction products can be purified by the

means described herein, or by other means known in the art.

EXAMPLE 1

(4R)-4-(t-Butyldimethylsilyloxy)cyclopent-2-enone (2; $R=SiBu^tMe_2$)

The (4R)-enone (6; $R=SiBu^tMe_2$) (125mg, 0.5 mmol) in methanol (3 ml) was added rapidly with vigorous stirring to zinc-silver couple freshly prepared from zinc (700mg) (R.D. Clark and C.H. Heathcock, J. Org. Chem., 1973, 38, 3658). After 5 min. the suspension was filtered through Celite, the residue washed with methanol (5 x 2ml), and the combined methanol solutions were evaporated under reduced pressure. Purification of the residue by preparative layer chromatography on silica gel in methylene dichloride - methanol (50:1) gave a colourless oil (102 mg) which crystallised from pentane (-78°) to yield the pure (4R)-enone (2; $R=SiBu^tMe_2$) (88mg, 83%) as colourless needles, m.p. 30-31°, $[\alpha]_D^{22}$ + 67° (c 0.117, MeOH), $[\alpha]_{348}^{25}$- 1660° (c $1.17 \times 10^{-2}$, MeOH), $[\alpha]_{235}^{25}$ + 16,830° (c $1.17 \times 10^{-3}$, MeOH), $[\theta]_{317}^{25}$ - 10,680 (c $1.17 \times 10^{-2}$, MeOH), $[\theta]_{217}^{25}$ + 77,810 (c $1.17 \times 10^{-3}$, MeOH) (Found : C, 62.05; H, 9.25. $C_{11}H_{20}O_2Si$ requires C, 62.2; H, 9.5%).

EXAMPLE 2

(4R)-4-Hydroxycyclopent-2-enone (2; R=H)

The (4R)-ether (2; $R=SiBu^tMe_2$) (130 mg, 0.61 mmol) in acetic acid-tetrahydrofuran-water (3:1:1, 5 ml) was maintained at room temperature for 24 h. Solvent was removed under reduced pressure, and the residue was chromatographed on preparative layers of silica gel with methylene dichloride-methanol (20:1) as eluant. The (4R)-enone (2; R=H) (55 mg, 92%) was obtained as a colourless oil, b.p. 45° / 0.05 mm.Hg, $[\alpha]_D^{22}$+ 81° (c 0.104, $CHCl_3$), $[\alpha]_D^{22}$+ 96° (c 0.112, MeOH), $[\alpha]_{348}^{25}$- 2765° (c $1.12 \times 10^{-2}$, MeOH), $[\alpha]_{231}^{25}$ + 33,910° (c $1.12 \times 10^{-3}$, MeOH), $[\theta]_{317}^{25}$ -7990

(c 1.12 x $10^{-2}$, MeOH), $[\Theta]^{25}_{215}$ + 68,020 (c 1.12 x $10^{-3}$, MeOH) (Found : C, 61.05; H, 6.25. $C_5H_6O_2$ requires C, 61.2; H, 6.15%).

EXAMPLE 3

(4R)-4-Acetoxycyclopent-2-enone (2; R=Ac).

The (4R)-alcohol (2; R=H) (40mg, 0.4 mmol), acetic anhydride (125mg, 1.22 mmol) and anhydrous sodium acetate (150mg, 1.8 mmol) were heated in tetrahydrofuran (3 ml) at reflux for 16 h. The mixture was cooled, diluted with water (3 ml) and extracted with ether (3 x 10 ml), and the combined ether extracts were washed with aqueous sodium chloride solution, dried ($MgSO_4$) and evaporated. Distillation (Kugelrohr, b.p. 45°/0.05 mm Hg) of the residue gave (4R)-4-acetoxycyclopent-2-enone (2; R=Ac) (48 mg, 86%) as an oil $[\alpha]^{22}_{D}$ + 97° (c 0.10, MeOH), $[\alpha]^{25}_{348}$ - 1355° (c 1.03 x $10^{-2}$, MeOH), $[\alpha]^{25}_{221}$ + 30,000° (c 1.03 x $10^{-3}$, MeOH), $[\Theta]^{25}_{320}$ - 5965 (c 1.03 x $10^{-2}$, MeOH), $[\Theta]^{25}_{208}$ + 80,010 (c 1.03 x $10^{-3}$, MeOH) (Found : C,60.15; H, 5.95. $C_7H_8O_3$ requires C, 60.0; H, 5.75%).

EXAMPLE 4

(4S)-4-Benzoyloxycyclopent-2-enone (3; R=PhCO) from (4R)-4-Hydroxycyclopent-2-enone (2; R=H).

The (4R)-alcohol (2;R=H)(30mg, 0.3 mmol), diethyl azodicarboxylate (47.3 μl, 0.6 mmol), triphenyl phosphine(78.8 mg, 0.6 mmol) and benzoic acid (36.6 mg, 0.6 mmol) were stirred together at room temperature in tetrahydrofuran (1 ml) for 20 h. Evaporation of the solvent under reduced pressure gave a semicrystalline residue which was triturated with ether. Filtration, removal of the solvent from the filtrate and preparative layer chromatography of the residual oil on silica gel in methylene dichloride-methanol (50:1) gave the (4S)-benzoate (3; R=PhCO) (40 mg, 66%) as a colourless oil, $[\Theta]^{25}_{322}$ + 5350 (c 7.1 x $10^{-2}$, MeOH) (Found :

C, 71.05; H, 5.25. $C_{12}H_{10}O_3$ requires C, 71.3; H, 5.0%).

EXAMPLE 5

(4S)-4-(t-Butyldimethylsilyloxy)cyclopent-2-enone (3; R=$SiBu^tMe_2$).

Obtained in 91% yield by a process analogous to Example 1 from the (4S)-enone (5; R=$SiBu^tMe_2$) as needles, m.p. 29-31° (from pentane at -78°), $[\alpha]_D^{22}$ -66° (c 5.01 x $10^{-2}$, MeOH), $[\alpha]_{348}^{25}$ + 1570° (c 5.01 x $10^{-2}$, MeOH), $[\alpha]_{235}^{25}$ -17,980° (c 1.00 x $10^{-3}$, MeOH), $[\Theta]_{317}^{25}$ + 10,100 (c 5.01 x $10^{-2}$, MeOH), $[\Theta]_{217}^{25}$ - 80,600 (c 1.00 x $10^{-3}$, MeOH) (Found : C, 62.2; H, 9.4. $C_{11}H_{20}O_2Si$ requires C, 62.2; H, 9.5%).

EXAMPLE 6

(4S)-4-Hydroxycyclopent-2-enone (3; R=H)

Obtained by a process analogous to Example 2 from the (4S)-enone (3; R=$SiBu^tMe_2$) as a colourless oil. This product showed similar behaviour on thin layer chromatography (silica gel, methylene dichloride-methanol, 10:1) and an identical 'H n.m.r. spectrum to the fully characterised (4R)-enantiomer (2; R=H) described in Example 2.

EXAMPLE 7

(4S)-4-Benzoyloxycyclopent-2-enone (3; R=PhCO) from (4S)-4-Hydroxycyclopent-2-enone (3; R=H).

To (4S)-alcohol (3; R=H) ( 16 mg, 0.16 mmol) in tetrahydrofuran (0.5 ml) at 0° were added pyridine (26 µℓ, 0.32 mmol) and benzoyl chloride(37µℓ, 0.32 mmol). After stirring at room temperature for 24 h, water (5 ml) was added and the mixture was extracted with ether (3x5 ml). The combined ether extracts were washed successively with saturated aqueous sodium chloride and water and the dried ($MgSO_4$) solvent was removed under reduced pressure. Chromatographic purification of the residue (55mg) on silica gel in methylene dichloride-

methanol (50:1) gave the (4S)- benzoate (3; R=PhCO) (24.2 mg, 75%), as an oil $[\theta]^{25}_{320}$ + 4910 (c 0.11,MeOH), with identical spectra to material obtained in Example 4.

It will, of course, be appreciated that the above examples are given by way of exemplification of the invention only, and that changes may be made to the details set out therein without departing from the scope of the invention.

CLAIMS

1. A process for the preparation of a compound of the general formula I:

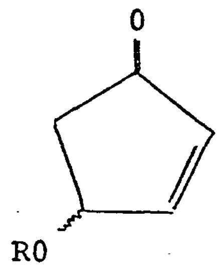

I

wherein R represents hydrogen or a protecting group, which comprises selective reduction of a compound of the general formula II:

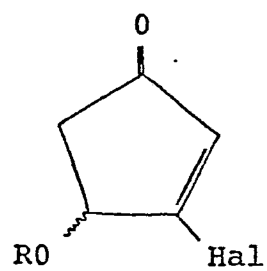

II

wherein R is as defined above and Hal represents halogen and, optionally, total or partial inversion of the hydroxyl or protected hydroxyl substituent, and, when R represents a protecting group, optionally, removal of the protecting group from the compound of general formula I.

2. A process as claimed in claim 1 wherein the protecting group is a substituted alkyl group, preferably an alkoxyalkyl group, a tetrahydrofuran-2-yl group or a tetrahydropyran-2-yl group, or a substituted silyl group, preferably a silyl group trisubstituted with alkyl and/or aryl residues, advantageously a t-butyldimethysilyl group.

3. A process as claimed in claim 1 wherein the protecting group is derived from an alkyl or aryl carboxylic acid, and is preferably an acetyl or benzyl group.

4. A process as claimed in any one of claims 1 to 3 wherein Hal represents a chloro, bromo or iodo group.

5. A process as claimed in any one of claims 1 to 4 wherein said selective reduction is carried out in the presence of a bimetallic couple, preferably a zinc-silver couple.

6. A process as claimed in any one of claims 1 to 5 including the further step of total or partial resolution of a racemic mixture of a compound of the general formula I.

7. A process as claimed in any one of claims 1 to 6, wherein the compound of the general formula II is prepared by partial dehalogenation of a compound of the general formula III:

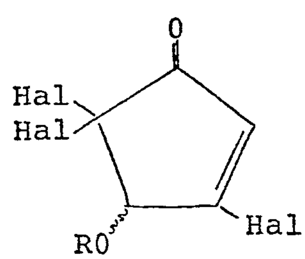

III

8. Compounds of the general formula I defined in claim 1, whenever prepared by a process according to any one of claims 1 to 7.

9. (4R)- and (4S)-4-(t-Butyldimethylsilyloxy) cyclopent-2-enone and all mixtures thereof, whenever prepared by a process according to any one of claims 1 to 7.

10. (4*R*)- and (4*S*)-4-Hydroxycyclopent-2-enone and all mixtures thereof, whenever prepared by a process according to claims 1 to 7.

0013620

European Patent Office

# EUROPEAN SEARCH REPORT

Application number
EP 80 30 0080

| Category | Citation of document with indication, where appropriate, of relevant passages | Relevant to claim | CLASSIFICATION OF THE APPLICATION (Int. Cl.) |
|---|---|---|---|
| | INTERNATIONAL JOURNAL OF METHODS IN SYNTHETIC ORGANIC CHEMISTRY, (1974), no. 8, page 586, no.3855. "Reduction of β-halo-α,β unsaturated cyclic ketones". * Abstract 3855 * | 1,5,4 | C 07 C 45/65<br>49/707<br>45/67<br>C 07 F 7/18//<br>C 07 C 69/145<br>69/78 |
| X | FR - A - 2 324 608 (TEIJIN) * Pages 72-73, examples 55,56 * | 1,2 | |
| D | & US - A - 4 132 726 | | |
| | | | TECHNICAL FIELDS SEARCHED (Int.Cl) |
| P | TETRAHEDRON LETTERS, April 1979, no. 17, pages 1539-41. Pergamon Press, Great Britain M. GILL et al. "Cyclopentanoids from phenol. Part III. Synthesis of chiral 4-hydroxycyclopent-2-enones". * Page 1540, compounds 1,2,3,5; page 1541 * | 1,2,4,5,9,10 | C 07 C 45/65<br>45/67<br>49/707<br>C 07 F 7/18<br>C 07 C 69/145<br>69/78 |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 17-04-1980 | Mme. BONNEVALLE |